# DEMANDE DE BREVET EUROPEEN

(11) **EP 1 593 365 A1**
(43) Date de publication de la demande: **09.11.2005**
(21) Numéro de dépôt: 05290541.1
(22) Date de dépôt: 10.03.2005
(51) Int. Cl.: A61K 7/00

(54) **Procédé non thérapeutique de traçabilité d'une composition contenant un composé enrichi en isotope stable non radioactif**

(30) Priorité: 12.03.2004 FR 0450511
(71) Demandeur: L'OREAL, 75008 Paris (FR)
(72) Inventeur: Saint-Leger, Didier, 92110 Clichy (FR)
(74) Mandataire: Fevrier, Murielle Françoise E.

(57) **Abrégé**

La présente invention se rapporte à un procédé non thérapeutique de traçabilité d'au moins un composé constitué d'au moins un élément atomique enrichi en au moins un isotope stable non radioactif, le dit composé étant contenu dans une composition cosmétique à usage topique comprenant un milieu physiologiquement acceptable, étant entendu que ledit isotope stable non radioactif enrichi est choisi parmi les isotopes naturels d'un élément atomique possédant au moins deux isotopes naturels stables non radioactifs, consistant à appliquer topiquement ladite composition sur les matières kératiniques humaines et/ou les muqueuses et à étudier la transformation et/ou le devenir dudit composé au sein des structures constitutives de ces matières kératiniques et/ou de ces muqueuses par prélèvement de ces matières kératiniques et/ou de ces muqueuses puis analyse de ce prélèvements pour quantifier l'isotope stable non radioactif présent dans ces matières kératiniques et/ou ces muqueuses.

## Description

La présente invention se rapporte à un procédé non thérapeutique de tracabilité, permettant le suivi d'un composé constitué d'au moins un élément atomique enrichi en isotope stable non radioactif, suite à l'application topique sur les matières kératiniques humaines et/ou les muqueuses telles que les lèvres du visage, d'une composition cosmétique contenant ce composé. L'isotope stable non radioactif enrichi est choisi parmi les isotopes naturels d'un élément atomique possédant au moins deux isotopes naturels stables non radioactifs. L'invention se rapporte encore à l'utilisation cosmétique d'un composé constitué d'au moins un élément atomique enrichi en isotope stable non radioactif dans une composition cosmétique à usage topique comprenant un milieu physiologiquement, comme agent pour étudier la transformation de cette composition et/ou son devenir dans les matières kératiniques humaines et/ou les muqueuses, après son application sur ces matières kératiniques et/ou ces muqueuses.

Par "matières kératiniques humaines", on entend au sens de l'invention, toute partie externe du corps humain contenant de la kératine comme la peau, y compris le cuir chevelu, les fibres kératiniques et en particulier les cheveux, les cils, les sourcils, les poils en particulier de barbe, ainsi que les ongles. Par "muqueuses", on entend principalement les lèvres du visage.

Il existe déjà de nombreuses méthodes de mesures permettant de connaître le devenir métabolique d'une molécule suite à son administration chez l'homme ou l'animal.
Dans ce domaine; l'utilisation d'isotopes radioactifs ou radio-isotopes est bien connue, en particulier en biologie dans des méthodes *in vitro,* ou *in vivo* chez l'animal. Les radio-isotopes fréquemment utilisés comme traceurs sont notamment l'hydrogène 3 (³H), le carbone 14 (¹⁴C), le phosphore 32 (³²P), le soufre 35 (³⁵S), l'iode 125 (¹²⁵I). L'utilisation de ces traceurs isotopiques radioactifs constitue une méthode puissante d'étude du métabolisme. Chaque molécule marquée par un radio-isotope n'est pas distinguable chimiquement d'une molécule normale non marquée, mais peut être facilement détectée et mesurée par sa radioactivité.

Le suivi des différentes transformations et/ou modifications d'une molécule est bien souvent difficile d'un point de vue technique chez l'homme en situation réelle, *in vivo.* Les techniques de prélèvements puis de dosages sont en effet limitant, tant par leur aspect invasif et toxique que par leur faible spécificité de dosages. En particulier, les composés dont les radio-isotopes constitutifs présentent des périodes de demi-vie faibles ou très faibles doivent être préparés juste avant emploi et sont difficiles à doser compte tenu de leur aspect évolutif, ce qui est le cas par exemple pour les composés contenant le phosphore 35. Se rajoutent de plus, extraits lors du prélèvement, des éléments naturellement constitutifs de la peau et/ou des cheveux qui peuvent interférer au cours du dosage, voire le fausser par des phénomènes tels que le "quenching" de ces isotopes radioactifs.

De plus, force est de reconnaître que l'utilisation de radio-isotopes présente chez l'homme des risques sécuritaires évidents eu égard à la nocivité potentielle des radiations émises. L'utilisation de radio-isotopes impliquant des règles d'utilisation et de manipulation strictes, on comprend aisément que des méthodes alternatives exemptes de radio-isotopes aient été recherchées de manière à pouvoir être mise en oeuvre en toute sécurité chez l'homme.

Dans le domaine des diagnostiques médicaux on connaît déjà des méthodes utilisant des isotopes stables non radioactifs. D'un point de vue sécuritaire, ces méthodes offrent, à l'inverse de celles utilisant des éléments atomiques radio-marqués, toute garantie d'absence d'effets secondaires, ces éléments atomiques n'étant nullement générateurs d'émissions radioactives, comme leurs homologues naturels les plus abondants (¹²C, ¹⁴N, ¹H, ...).

On peut citer par exemple des méthodes de mesure du taux de renouvellement ("turnover") du glucose, de l'alanine et de la gluconéogenèse comprenant l'injection de D-[2,3,4,6,6-²H 5]glucose et de L-[1,2,3-¹³C 3]alanine et des analyses par GC-MS (Chromatographie gazeuse couplée à la spectrométrie de masse) (Martineau A. *et al*., Anal Biochem., 1985, Dec; 151(2):495-503).

On connaît également des études sur la biodisponibilité du glucose plasmatique utilisant l'ingestion d'aliments à base d'amidon enrichi en ¹³C et leur analyse par spectrométrie de masse (Norman S. *et al*., Am. J. Clin. Nutr., 1992 Feb; 55(2):430-5). On peut encore citer une méthode permettant de caractériser des maladies respiratoires via l'émission de ¹⁵NO, suite à l'inhalation de [¹⁵N₂] Arginine comme agent de diagnostique (P. Krumbiegel *et al*., Experimental Lung Research 28:535-542, 2002).

Si les utilisations par voie orale, pulmonaire ou systémique des isotopes stables telles que rapportés dans l'art antérieur permettent à l'homme de l'art de mieux connaître le devenir d'une molécule donnée et ses différentes transformations par un organe donné, elles sont peu ou pas transposables aux matières kératiniques humaines telles que la peau (y compris le cuir chevelu) et les fibres kératiniques (cheveux, cils, poils et/ou ongles) et/ou aux muqueuses (lèvres du visage notamment). En effet, l'administration par voie orale, par voie pulmonaire ou par voie systémique d'une molécule va conduire, après transformation, notamment par l'intestin et/ou le foie, à la genèse de dérivés qui ne correspondent plus à la molécule de départ. Ces transformations sont variables suivant les organes considérés et leurs analyses nécessitent des méthodes invasives et/ou destructrices et/ou des méthodes globales de dosage (urine, etc...), ces dernières ne permettant pas, en outre de savoir où s'est effectuée la transformation. Dés lors, l'étude de la transformation de cette molécule, spécifiquement au niveau des matières kératiniques et/ou des muqueuses, devient difficile sinon impossible.

Les produits appliqués sur les matières kératiniques humaines ou les lèvres, qu'ils soient d'ordre cosmétique (apparat, confort, protection, soin..) ou dermatologique (traitement, cure..) nécessitent bien souvent d'être suivis dans leur devenir, une fois appliqués, que ce soit pour des raisons de mise en évidence de leur efficacité ou pour des raisons de leur sécurité. Suite à leur application topique sur les matières kératiniques humaines ou les muqueuses, ces produits peuvent en effet, subir différents évènements comme, par exemple, leur bio-transformation par les matières kératiniques, leur élimination par lavage (savon, douche) ou par voie naturelle (sueur...), leur transformation structurelle par différentes radiations solaires visibles ou non (UV), etc....

En outre, afin de déterminer la concentration optimale d'un produit devant être appliqué sur les matières kératiniques ou les muqueuses, des études préalables sont nécessaires afin de connaître son suivi après application.

Bien que les isotopes stables existent et soient utilisés dans le domaine du diagnostique médical depuis de nombreuses années, une méthode non thérapeutique de traçabilité permettant notamment le suivi d'un composé constitué d'au moins un élément atomique enrichi en isotope stable suite à l'application topique sur les matières kératiniques ou les muqueuses, humaines, d'une composition topique le contenant n'a, à la connaissance de la demanderesse, encore jamais été décrite dans l'art antérieur.

La présente invention se rapporte donc à un procédé non thérapeutique de traçabilité, permettent le suivi d'au moins un composé constitué d'au moins un élément atomique enrichi en au moins un isotope stable non radioactif suite à l'application topique sur les matières kératiniques ou les muqueuses, humaines, d'une composition cosmétique à usage topique contenant, dans un milieu physiologiquement acceptable, au moins ledit composé constitué d'au moins un élément atomique enrichi en au moins un isotope stable non radio-actif, étant entendu que le dit isotope stable non radioactif est choisi parmi les isotopes naturels d'un élément atomique possédant au moins deux isotopes naturels stables non radioactifs.

De façon plus précise, l'invention a pour objet un procédé non thérapeutique de traçabilité d'au moins un composé constitué d'au moins un élément atomique enrichi en au moins un isotope stable non radioactif, le dit composé étant contenu dans une composition cosmétique à usage topique comprenant un milieu physiologiquement acceptable, étant entendu que ledit isotope stable non radioactif enrichi est choisi parmi les isotopes naturels d'un élément atomique possédant au moins deux isotopes naturels stables non radioactifs, consistant à appliquer ladite composition sur les matières kératiniques humaines et/ou les muqueuses et à étudier la transformation et/ou le devenir dudit composé au sein des structures constitutives de ces matières kératiniques et/ou de ces muqueuses par prélèvement de ces matières kératiniques et/ou de ces muqueuses puis analyse de ce prélèvement pour quantifier l'isotope stable non radioactif présent dans ces matières kératiniques et/ou ces muqueuses.

Si nécessaire, le procédé comprend une étape de préparation du prélèvement en vue de son analyse telle qu'une étape d'extraction de cellules puis de séparation de ces cellules par exemple par chromatographie, une étape d'élution, un étape d'hydrolyse puis éventuellement de séparation par exemple par chromatographie,

De préférence, l'isotope naturel n'est pas celui majoritaire du dit élément atomique si la teneur dudit isotope majoritaire est supérieure à 50% dans l'élément atomique naturel.

L'isotope stable non radioactif est donc avantageusement choisi parmi le ou les isotopes naturels minoritaires dudit l'élément atomique.

Les compositions qui sont utilisées dans le procédé non thérapeutique de traçabilité selon l'invention sont caractérisées en ce que le pourcentage d'au moins un isotope stable non radioactif total d'au moins un élément atomique présent dans au moins un composé de la composition est supérieur au pourcentage isotopique naturel. Avantageusement, le pourcentage isotopique de l'isotope enrichi dans ledit composé considéré varie de 0,00014 à 100%. Le composé peut donc ne contenir que l'isotope considéré. Avantageusement, l'enrichissement en au moins un isotope stable non radioactif d'au moins un élément atomique dans au moins un composé de la composition se traduit au niveau du composé par un rapport teneur finale de l'isotope /teneur naturelle qui varie de 1,01 à 10⁶, de préférence de 1,02 à 10000, très préférentiellement de 1,05 à 5000.

Par élément atomique au sens de la présente invention on entend un élément atomique compris dans la classification périodique des éléments chimiques.

Par matières kératiniques au sens de la présente invention on entend la peau, y compris le cuir chevelu, et/ou les fibres kératiniques telles que les cheveux, les cils, les sourcils, les poils, les ongles, plus spécialement les cheveux.

Les isotopes stables non radioactifs, qui peuvent être utilisés dans le procédé suivant l'invention, sont de préférence choisis parmi les éléments atomiques dont le numéro atomique varie de 1 à 70, avantageusement de 1 à 50.

De façon particulièrement préférée les isotopes stables non radioactifs sont choisis parmi le deutérium (²D ou ²H, isotope de l'Hydrogène), l'hélium 3 (³He), le lithium 6 (⁶Li), le bore 10 (¹⁰B), le carbone 13 (¹³C), l'azote 15 (¹⁵N), l'oxygène 17 (¹⁷O), l'oxygène 18 (¹⁸O), le magnésium 25 (²⁵Mg), le magnésium 26 (²⁶Mg), le silicium 29 (²⁹Si), le silicium 30 (³⁰Si), le soufre 33 (³³S), le soufre 34 (³⁴S), le soufre 36 (³⁶S), le chlore 37 (³⁷Cl), le potassium 40 (⁴⁰P), le potassium 41 (⁴¹P), le calcium 42 (⁴²Ca), le calcium 43 (⁴³Ca), le calcium 44 (⁴⁴Ca), le calcium 46 (⁴⁶Ca), le calcium 48 (⁴⁸Ca), le fer 56 (⁵⁶Fe), le fer 57 (⁵⁷Fe), le fer 58 (⁵⁸Fe), le cuivre 65 (⁶⁵Cu), le zinc 66 (⁶⁶Zn), le zinc 67 (⁶⁷Zn), le zinc 68 (⁶⁸Zn), le zinc 70 (⁷⁰Zn), le strontium 84 (⁸⁴Sr), le strontium 87 (⁸⁷Sr), le strontium 84 (⁸⁴Sr). Avantageusement, l'isotope stable non radioactif est choisi parmi le deutérium, le carbone 13, l'azote 15, l'oxygène 17, l'oxygène 18, le soufre 34.

Selon l'invention, on incorpore au moins un isotope stable dans au moins un composé (ou molécule) dont on souhaite étudier le suivi ou le devenir après l'application topique d'une composition le contenant, grâce à la mise en oeuvre du procédé non thérapeutique suivant l'invention.

Au sens de la présente invention, on entend par "suivi" ou "devenir" toute information (adsorption, distribution, métabolisation, excrétion) concernant un composé ou une molécule, y compris sa transformation, sa biodisponibilité, sa présence ou non et son taux d'incorporation au sein des matières kératiniques humaines et ses annexes, notamment les glandes sébacée et sudorale ou des muqueuses humaines.

Ainsi, l'application topique suivant l'invention d'une composition cosmétique contenant au moins un composé dont les éléments atomiques constitutifs sont totalement ou partiellement composés d'isotopes stables non radioactifs, conduira à enrichir la zone d'application en isotopes de cet élément atomique, enrichissement qui sera ensuite déterminé quantitativement et qualitativement par les moyens analytiques appropriés, couplés ou non à des procédures de séparation chromatographiques préalables, comme par exemple, la RMN (Résonance Magnétique Nucléaire) ou la spectrométrie de masse IR-MS (Isotope Ratio-Mass Spectrometry), la SIMS (Secondary Ion Mass Spectroscopy). Ces techniques sont bien connues de l'homme du métier.

Les isotopes stables non radioactifs selon l'invention sont présents naturellement au sein de la matière organique, à de faibles mais constantes concentrations. A titre d'exemple on peut citer, le ¹³C qui représente 1,1% des atomes de carbone naturellement présents au sein des organismes vivants. Dés lors, l'application d'un composé cosmétique (ou molécule cosmétique) dont la ou les chaînes carbonées sont par exemple uniquement composées de ¹³C, conduira à enrichir la zone d'application en cet élément atomique, enrichissement (passage de 1.1% à 1.2% ou 1.5% du contenu ¹³C/Carbone total) qui sera ensuite déterminé quantitativement et qualitativement par les moyens analytiques cités précédemment. On peut encore citer le deutérium, l'azote 15, l'oxygène 17, l'oxygène 18, le soufre 34, qui représentent respectivement 0.015%, 0.365%, 0.037%, 0.204% et 4.215% des atomes de carbone naturellement présents au sein des organismes vivants.

La mise en oeuvre du procédé suivant l'invention consistant à appliquer topiquement une composition contenant au moins un composé (ou molécule) constitué d'au moins un élément atomique enrichi en au moins un isotope stable non radioactif sur les matières kératiniques (peau -y compris le cuir chevelu- et/ou les fibres kératiniques) et/ou sur les muqueuses (lèvres du visage), et notamment les cheveux permet alors, selon le but recherché, l'étude de la transformation et/ou du devenir dudit composé au sein des structures constitutives desdites matières kératiniques.

Avantageusement, le procédé suivant l'invention consiste:
- à suivre le devenir et/ou les modifications d'un composé suite à son application topique sur les matières kératiniques ou les muqueuses ou,
- à mesurer l'absence de pénétration trans-cutanée ou trans-cuticulaire dudit composé, suite à son application topique sur les matières kératiniques comme la peau ou les cheveux ou sur les muqueuses ou,
- à suivre dans l'environnement le devenir dudit composé suite à son élimination de l'organisme par lavage (ou nettoyage) des matières kératiniques et/ou des muqueuses.

Le composé constitué d'au moins un élément atomique enrichi en au moins un isotope stable non radioactif est choisi parmi un solvant hydrosoluble, de préférence l'éthanol; une silicone; un colorant capillaire, de préférence un colorant direct, un pigment minéral, organique ou nacré; un précurseur de colorants d'oxydations; un coupleur ou modificateur de coloration; un conservateur; un polymère, de préférence un polymère filmogène; un tensioactif; un agent défrisant ou de permanente; un agent oxydant, de préférence l'eau oxygénée; un agent réducteur; un actif cosmétique comme un filtre UV, un agent hydratant, un agent pigmentant cutané, un agent astringent, un agent antipelliculaire.

Le procédé suivant l'invention peut notamment trouver les applications suivantes :

### 1) Suivi de l'évolution d'un précurseur de coloration capillaire

La Para Phénylène Diamine (PPD) qui, obtenue par synthèse, pourra comporter soit six atomes de carbone sous forme ¹³C et/ou deux atomes d'azote sous forme ¹⁵N. L'application d'une formule colorante, à base de cette PPD pourra être ensuite menée *in vivo* chez l'homme. Le prélèvement de quelques cheveux, post coloration, permettra à l'homme de l'art de suivre l'évolution des pigments ainsi formés, leur élimination, leur localisation au sein des cheveux, à différents temps ou selon différents traitements (lavages, irradiation lumineuse... ) ou procédures, notamment celles décrites dans l'ouvrage « The Science of Hair Care, C Zviak, Marcel Dekker, New York , 1986).

### 2) Suivi d'un composé naturel sur le cuir chevelu

L'ester de Glucose et d'acide linoléique (liaison ester sur la position 6 du Glucose), peut être synthétisé à partir de composés Glucose et acide linoléique dont tous les atomes de Carbone sont sous forme ¹³C (disponibles commercialement, Société Isotec, Sigma Aldrich/USA).
L'application de manière répétée de cet ester sur le cuir chevelu de l'homme *in vivo*, pourra alors permettre de suivre sa transformation, sa dégradation éventuelle, sa biodisponibilité vis à vis des structures comme le follicule pileux, la glande sébacée, sa réutilisation selon les voies métaboliques classiques, etc...
Suite à des applications successives du dit ester sur le cuir chevelu humain, *in vivo*, il est alors possible de prélever chez les personnes, quelques cheveux dont l'extrémité contient le plus souvent la partie « vivante » du follicule pileux, en l'occurrence une gaine dite externe composée de plusieurs centaines de cellules. L'extraction de ces cellules peut être ensuite réalisée et les différentes molécules présentes peuvent ensuite être séparées par un moyen chromatographique couplé à la Spectrométrie de masse (dite GC-MS par exemple). Les quantités résiduelles de ¹³C sont alors détectées, ce qui permet de déterminer par quels chemins métaboliques, le composé ester initial a été transformé ou dégradé.

### 3) Mesure de la capacité d'une formule cosmétique à véhiculer le Glycérol, à des fins d'hydratation cutanée

Le Glycérol, introduit à des doses variables, de 2 à 10 %, est un excellent hydratant cutané, connu depuis longtemps et utilisé le plus souvent afin de lutter contre la peau sèche. Son action, au sein de la couche cornée humaine est entre autre, liée à sa bonne diffusion/pénétration au sein des couches épidermiques les plus profondes (Taylor L.J. *et al*., Effect of occlusion on the percutaneous penetration of linoleic acid and glycerol, Int. J. Pharm, 5-249, 157-164, 2002).
Si sa formulation ne pose guère de difficultés, encore est-il nécessaire, pour l'homme de l'art, de pouvoir sélectionner les formules les plus aptes à faire diffuser le dit glycérol. L'utilisation, *in vivo*, de Glycérol marqué sur ses 3 atomes de Carbone sous forme ¹³C (disponible commercialement chez la Sté Isotec sous la référence 48,947-6) est ainsi un moyen de quantifier, *in vivo* ce critère. Après des applications de différentes crèmes cosmétiques (de compositions différentes mais toutes formulées avec par exemple 4.9% de ¹²C Glycérol et 0.1% de ¹³C Glycérol) sur l'avant bras de sujets humains, il est possible d'effectuer une série de "strippings" (arrachages ou prélèvements discrets et successifs de couches comées cutanées par des bandelettes adhésives type « Scotch® » ou « Blenderm® »). Suivant des techniques bien connues des l'homme du métier, ces "strippings" sont alors ensuite analysés par Spectrométrie de Masse qui permet alors de quantifier la quantité de ¹³C présente sur chaque "stripping" et de déterminer enfin la crème qui a véhiculé le plus de Glycérol au sein des différentes strates. Selon la technique d'analyse disponible ou choisie, les "strippings" ainsi obtenus pourront être directement analysés par exemple à l'aide de la technique SIMS ou encore élués à l'aide de solvants (l'eau ou toute solution aqueuse dans le cas du Glycérol) appropriés à la nature des composés ou molécules permettant ensuite leur analyse en milieu liquide en spectrométrie de Masse.

### 4) Détermination de la présence et du taux d'incorporation d'un acide aminé au sein du follicule pileux

Des acides aminés contenant un ou plusieurs ¹³C et/ou ¹⁵N (Acide Glutamique, Cystéine,..), sont appliqués par voie topique sur le cuir chevelu d'un homme *in vivo.* Sur des temps assez longs (plusieurs semaines) les acides aminés sont réutilisés par le follicule pileux dans l'élaboration des chaînes kératiniques qui constituent l'essentiel de la fibre capillaire. Suite au prélèvement de quelques cheveux et leur hydrolyse en milieu alcalin ou enzymatique, les acides aminés des chaînes kératiniques sont chromatographiés et une analyse par RMN permet ensuite de déterminer leur présence et le taux d'incorporation du ¹³C et/ou du ¹⁵N.

### 5) Etude de la rémanence d'agents lavant tensioactifs.

Une solution lavante peut ainsi être élaborée par le mélange, dans l'eau, de 4.9% de Sodium ¹²C Lauryl Sulfate et de 0.1% de Sodium 25D Lauryl Sulfate (Isotec 45,185-1) où tous les 25 atomes d'Hydrogène sont des atomes de Deutérium, en présence ou non d'agents divers tels que des polymères, des parfums, des colorants. Appliquée sur les avant-bras de quelques sujets, à des fins de simulation de lavage, les solutions lavantes sont ensuite rincées abondamment sous l'eau puis séchés. Là encore, quelques "strippings" effectués sur la zone appliquée permettront, par leur analyse en Spectrométrie de Masse, de déterminer la fraction de Sodium Lauryl Sulfate encore éventuellement présente dans les couches les plus superficielles de la peau. Une telle procédure permet, par modifications successives de la formule d'appréhender le ou les constituants qui favoriseront ou non le rinçage du tensioactif considéré.

### 6) Suivi dans l'environnement de produits ou compositions cosmétiques après leur élimination par lavage

L'application topique des produits cosmétiques étant suivie de lavages quotidiens, le suivi (modifications, éliminations, cycle de vie) de leur devenir du point de vue environnemental (au sein des eaux résiduelles, dans la terre, par les micro organismes terrestres et les animaux inférieurs...) pourra ainsi être effectué sans aucune conséquence néfaste du point de vue écologique, évitant l'utilisation de molécules radioactives. Par exemple, le composé tracé peut être le tensioactif précédemment cité.

Avantageusement, les compositions utilisables dans l'invention sont telles que le pourcentage de l'isotope stable non radioactif enrichi total d'au moins un élément atomique présent dans un composé de ces compositions est supérieur au pourcentage isotopique naturel. Avantageusement, le pourcentage isotopique de l'isotope enrichi dans ledit composé considéré varie de 0,00014 à 100%. Le composé peut donc ne contenir que l'isotope considéré. Avantageusement, l'enrichissement dudit isotope stable non radioactif se traduit dans ledit composé par un rapport teneur finale de l'isotope / teneur naturelle qui varie de 1,01 à 10⁶, de préférence de 1,02 à 10000, très préférentiellement de 1,05 à 5000.

Les isotopes stables non radioactifs, préférés sont ceux qui ont été mentionnés précédemment. Avantageusement, l'isotope stable non radioactif est choisi parmi le deutérium, le carbone 13, l'azote 15, l'oxygène 17, l'oxygène 18, le soufre 34.

De préférence le dit composé constitué d'au moins un élément atomique enrichi en au moins un isotope stable non radioactif est choisi parmi un solvant hydrosoluble, de préférence l'éthanol; une silicone; un colorant capillaire, de préférence un colorant direct, un pigment minéral, organique ou nacré; un précurseur de colorants d'oxydations; un coupleur ou modificateur de coloration; un conservateur; un polymère, de préférence un polymère filmogène; un tensioactif; un agent défrisant ou de permanente; un agent oxydant, de préférence l'eau oxygénée; un agent réducteur; un actif cosmétique comme un filtre UV, un agent hydratant, un agent pigmentant cutané, un agent astringent, un agent antipelliculaire.

L'invention se rapporte encore à l'utilisation cosmétique d'au moins un composé constitué d'au moins un élément atomique enrichi en au moins un isotope stable non radioactif tel que défini précédemment, pour la préparation d'une composition cosmétique à usage topique, en vue d'en étudier sa transformation eUou son devenir dans les matières kératiniques humaines et/ou les muqueuses, après son application sur ces matières kératiniques et/ou ces muqueuses.

La composition contenant au moins un composé constitué d'au moins un élément atomique enrichi en isotope stable non radio-actif comprend un milieu physiologiquement acceptable, c'est-à-dire compatible avec les matières kératiniques humaines et les muqueuses humaines et non toxique, et plus spécialement cosmétique, ce qui sous entend que ladite composition peut être utilisée quotidiennement, pendant plusieurs mois sans prescription médicale. Elle permet un d'améliorer l'esthétique des fibres kératiniques humaines et notamment des cheveux et des cils ainsi que l'esthétique de la peau en leur donnant une plus grande vigueur et/ou un aspect amélioré. Elle peut se présenter sous toutes les formes galéniques classiquement utilisées pour une application topique et notamment sous forme de gels aqueux, de solutions aqueuses ou hydroalcooliques. Elle peut aussi, par ajout d'une phase grasse ou huileuse, se présenter sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsion multiple (E/H/E ou H/E/H), de microémulsion, de dispersion vésiculaire de type ionique et/ou non ionique, ou de dispersion cire/phase aqueuse, de nano-émulsion. Cette composition est préparée selon les méthodes usuelles.

La composition utilisée selon l'invention peut être plus ou moins fluide et avoir l'aspect d'une crème blanche ou colorée, d'une pommade, d'un lait, d'une lotion, d'un sérum, d'une pâte, d'une mousse. Elle peut être éventuellement appliquée sur le contour de l'oeil sous forme solide, et par exemple sous forme coulée, en coupelle ou sous forme de stick.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme matières grasses utilisables dans la composition utilisée selon l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans la composition utilisée selon l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

La composition utilisée selon l'invention peut également contenir les adjuvants et/ou additifs hydrophiles ou lipophiles habituels dans le domaine cosmétique, avantageusement choisis parmi les gélifiants; les actifs notamment cosmétiques hydrophiles ou lipophiles ayant un effet bénéfique sur les matières kératiniques ou les muqueuses; les conservateurs; les antioxydants; les solvants; les parfums; les charges; les nacres; les filtres UV; les absorbeurs d'odeur; les eaux parfumées; les colorants directs, les pigments minéraux, organiques ou nacrés, les précurseurs de colorants tels que les bases d'oxydation ou les coupleurs; les oxydants; les réducteurs; les agents conditionneurs pour les cheveux tels que les polymères cationiques ou amphotères, les tensioactifs cationiques, les dérivés siliconés, les huiles végétales, les cires, les céramides; les agents tensioactifs moussants anioniques, non ioniques ou amphotères; les agents de mise en forme temporaire des cheveux tels que des polymères anioniques, cationiques ou non ioniques; les agents épaississants; les polymères filmogènes.

Les quantités de ces différents adjuvants et/ou additifs sont celles classiquement utilisées dans le domaine considéré, et par exemple de 0,01 à 20 % du poids total de la composition. Ces adjuvants et/ou additifs, selon leur nature, peuvent être introduits dans la phase grasse ou dans la phase aqueuse.

Comme actifs cosmétiques hydrophiles ou lipophiles ayant un effet bénéfique sur les matières kératiniques, utilisables dans la composition pour la mise en oeuvre du procédé, on peut citer notamment les agents hydratants; les agents dépigmentants ou propigmentants; les agents anti-pollution et/ou anti-radicalaire; les agents antimicrobiens; les inhibiteurs de NO-synthase; les agents stimulant la synthèse de macromolécules dermiques ou épidermiques et/ou empêchant leur dégradation; les agents stimulant la prolifération des fibroblastes ou des kératinocytes et/ou la différenciation des kératinocytes; les agents dermo-décontractants; les agents tenseurs; les agents apaisants; les agents agissant sur la microcirculation et/ou les agents agissant sur le métabolisme énergétique des cellules et leurs mélanges; les humectants comme la glycérine, le sorbitol, le pentaérythritol, l'acide pyrrolidone carboxylique et ses sels; les agents de brunissage artificiel tels que la dihydroxyacétone, l'érythrulose, le glycéraldéhyde, les gamma-dialdéhydes tels que l'aldéhyde tartrique; les antiperspirants; les déodorants; les astringents; les produits rafraîchissants, toniques, cicatrisants, kératolytiques, dépilatoires; les extraits de tissus végétaux, tels que les polysaccharides: les agents antipelliculaires; les agents antiséborrhéiques; les agents antipelliculaires; les agents antiséborrhéiques, des agents régulateurs de la pousse des cheveux.

Comme colorants directs, on peut citer les familles des anthraquinones, des dérivés nitrés, benzèniques et azoïques.

Comme pigments minéraux, organiques ou nacrés, on peut citer, les oxydes de zinc, de titane, les oxydes de fer, les laques organiques, le mica-titane, des dispersions de polystyrène, des agents nacrants tel que le distéarate d'Ethylène Glycol.

Comme précurseurs de colorants d'oxydations, on peut citer la para phénylène diamine, le paraminophénol.

Comme coupleurs ou modificateurs de coloration, on peut citer la métaphénylène diamine, le métaminophénol, la résorcine.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol contenant également un agent propulseur sous pression.

La composition utilisable selon l'invention peut notamment se présenter sous la forme :
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres, un vernis à ongles, un soin des ongles, un mascara, un mascara traitant, un eye-liner;
- d'une composition de protection solaire ou de bronzage artificiel;
- d'un gel ou d'une lotion après-rasage;
- d'une crème dépilatoire;
- d'une composition dermatologique;
- d'une composition solide telle qu'un savon ou un pain de nettoyage;
- d'une composition pour aérosol comprenant également un agent propulseur sous pression;
- d'une lotion de mise en plis, d'une crème ou d'un gel coiffant,
- d'une composition de teinture (notamment d'oxydation) éventuellement sous forme de shampooing colorant;
- d'une lotion restructurante pour cheveux, une composition de permanente, une lotion ou un gel antichute;
- d'une composition capillaire, notamment sous forme de crème;
- un gel protecteur solaire;
- une composition de soin du cuir chevelu, notamment anti-chute ou repousse des cheveux;
- d'une composition de lavage telle qu'un shampoing ou un produit de démaquillage;
- d'une composition après shampoing notamment démêlant;
- d'un produit de soin, de traitement ou de protection, des matières kératiniques, et notamment de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil.

L'invention sera maintenant illustrée par les exemples non limitatifs suivants.

### EXEMPLES

Les compositions ci-dessous sont préparées de manière classique pour l'homme du métier. Sauf exception, la nomenclature CTFA est utilisée pour désigner leurs ingrédients.

### Exemple 1 : Préparation colorante pour cheveux à base de ¹⁵N₂ PPD

Synthèse préalable de ¹⁵N₂ Para Phénylène Diamine (PPD ou 1,4 diaminobenzène) par simple réduction de la ¹⁵N₂ Para Nitro Aniline dont les 2 atomes d'azote sont sous forme ¹⁵N (commercialisée par la Société Isotec/Sigma Aldrich, référence 48,773-2).
Dans un hydrogénateur on place 5 grammes de ¹⁵N₂ para Nitroaniline, 8 g de palladium à 5% sur charbon actif (contenant 50% d'eau), 150 ml d'éthanol à 96° et 150 ml d'eau.
La réduction est faite en une demi-heure sous une pression d'hydrogène d'environ 8 bars et à une température qui a été progressivement portée à 75°C.
Après filtration du catalyseur (Pd) sous azote on évapore le filtrat à sec sous pression réduite.
Le composé cristallisé obtenu est ensuite purifié par recristallisation dans l'éthanol au reflux. On obtient ainsi une Paraphénylène Diamine dont les deux groupements azotés (NH₂) sont tous deux constitués par des isotopes stables de l'azote 15.

| Préparation colorante | |
|---|---|
| ¹⁵N₂ PPD (¹⁵N₂ 1,4- diaminobenzène) | 0,150 g |
| 1-Amino 4-hydroxybenzène | 0,050 g |
| 1,3-Dihydroxybenzène | 0,150 g |
| 1-Hydroxy 3- aminobenzène | 0,150 g |
| 2-N-Méthylamino 4 β-hydroxyéthyloxy niitrobenzène | 0,050 g |
| Tertiobutylhydroquinone | 0,166 g |
| Bisulfite de Sodium à 35° Bé | 1,500 g |
| Alcool oléique glycérolé à 2 moles de glycérol | 5,000 g |
| Alcool oléique glycérolé à 4 moles de glycérol | 5,000 g |
| Acide oléique | 5,000 g |
| Diéthanolamine oléique | 5,000 g |
| Diéthanolamide oléique | 12,000 g |
| Alcool éthylique | 10,000 g |
| Ethoxy-2 éthanol | 12,000 g |
| Acide éthylènediaminotétracétique | 0,200 g |
| Ammoniaque à 22° Bé | 10,200 g |
| Eau | qsp 100 g |

Cette composition est un liquide que l'on mélange au moment de l'emploi avec son poids d'eau oxygénée à 20 volumes. Le mélange gélifié obtenu est appliqué sur une chevelure châtain clair fraîchement permanentée. Après 30 minutes de pose, le produit est éliminé par rinçage. Les cheveux sont shampouinés et séchés. Ils sont alors colorés en blond doré nacré.
Le processus de coloration, impliquant le couplage de la ¹⁵Npara phénylène diamine avec les autres coupleurs, le pigment colorant obtenu est ainsi enrichi en azote 15.

On peut alors suivre la ¹⁵N₂ Para Phénylène Diamine dans le cheveu au cours du temps par détection de l'azote ¹⁵N par spectrométrie de masse après prélèvement du cheveu.

### Exemple 2 : Formule capillaire nutritive à base de 6-Octadécénoate de Glucose

La synthèse préliminaire du 6-Octadécénoate de Glucose peut être réalisée suivant le procédé de synthèse décrit dans le document EP1371658 à partir de l'acide ¹³C₁₈ linoléique (tous les 18 atomes de carbone étant sous forme ¹³C) et de D glucose ¹³C₆ (tous les 6 atomes de carbone étant sous forme ¹³C), les deux composés étant commercialement disponibles chez la Société Isotec/Sigma Aldrich. Les 24 atomes de carbone constitutifs de l'ester ainsi obtenu sont sous la forme ¹³C.

| | |
|---|---|
| ¹³C₂₄ -6 Octadécénoate de Glucose | 0,5 g |
| Huile de Ricin hydrogénée oxyéthylénée 40 | 0,5 g |
| Butylhydroxytoluène | 0,1 g |
| Ethanol | 50,7g |
| Parfum | qs |
| Eau | qsp 100g |

On peut alors suivre le ¹³C₂₄-6 Octadécénoate de Glucose dans le cheveu au cours du temps par détection du carbone ¹³C par spectrométrie de masse après prélèvement du cheveu.

### Exemple 3 : Composition lavante pour cheveux sous forme de Shampooing (en %,poids/poids)

| | |
|---|---|
| Laureth 12 | 0,250 |
| Sodium Laureth Sulfate | 7,400 |
| D₂₅Sodium Lauryl Sulfate (Isotec 45,185-1) | 1,000 |
| Sodium Methyl Paraben | 0,200 |
| Propylène Glycol | 0,450 |
| DMDM Hydantoïne | 0,110 |
| Cocamide MAE | 0,500 |
| Coco Bétaïne | 3,000 |
| Sodium Chloride | 1,650 |
| Parfum | 0,500 |
| Acid tallow 3 (colorant) | 0,002 |
| Eau | qsp 100 |

On peut alors suivre le D₂₅Sodium Lauryl Sulfate dans le cheveu au cours du temps par détection du deutérium par spectrométrie de masse après prélèvement du cheveu.

### Exemple 4 : Composition de Crème hydratante visage (en %, poids/poids)

| | |
|---|---|
| Cetyl alcool | 7,00 |
| ¹²C Glycérol | 5,30 |
| ¹³C₃ Glycérol (Isotec 48,947-6) | 2,00 |
| Mineral oil | 2,00 |
| PEG-50 Stéarate | 2,50 |
| Acide Stéarique | 0,13 |
| Glyceryl Stearate SE | 2,25 |
| Isopropyl Palmitate | 3,00 |
| Fragrance | 0,30 |
| Methyl Paraben (conservateur) | 0,30 |
| Eau | qsp 100 |

On peut alors suivre le ¹³C₃ Glycérol dans la peau au cours du temps par détection du carbone ¹³C par spectrométrie de masse après prélèvement par "stripping".

## Revendications

1. Procédé non thérapeutique de traçabilité d'au moins un composé constitué d'au moins un élément atomique enrichi en au moins un isotope stable non radioactif, le dit composé étant contenu dans une composition cosmétique à usage topique comprenant un milieu physiologiquement acceptable, étant entendu que ledit isotope stable non radioactif enrichi est choisi parmi les isotopes naturels d'un élément atomique possédant au moins deux isotopes naturels stables non radioactifs, consistant à appliquer topiquement ladite composition sur les matières kératiniques humaines et/ou les muqueuses et à étudier la transformation et/ou le devenir dudit composé au sein des structures constitutives de ces matières kératiniques et/ou de ces muqueuses par prélèvement de ces matières kératiniques et/ou de ces muqueuses puis analyse de ce prélèvements pour quantifier l'isotope stable non radioactif présent dans ces matières kératiniques et/ou ces muqueuses.

2. Procédé suivant la revendication précédente, **caractérisé en ce que** l'isotope stable non radioactif est choisi parmi le ou les isotopes naturels minoritaires dudit élément atomique.

3. Procédé suivant l'une quelconque des revendications précédentes, **caractérisé en ce que** l'isotope stable non radioactif est choisi parmi le deutérium (²D), l'hélium 3 (³He), le lithium 6 (⁶U), le bore 10 (¹⁰B), le carbone 13 (¹³C), l'azote 15 (¹⁵N), l'oxygène 17 (¹⁷O), l'oxygène 18 (¹⁸O), le magnésium 25 (²⁵Mg), le magnésium 26 (²⁶Mg), le silicium 29 (²⁹Si), le silicium 30 (³⁰Si), le soufre 33 (³³S), le soufre 34 (³⁴S), le soufre 36 (³⁶S), le chlore 37 (³⁷Cl), le potassium 40 (⁴⁰P), le potassium 41 (⁴¹P), le calcium 42 (⁴²Ca), le calcium 43 (⁴³Ca), le calcium 44 (⁴⁴Ca), le calcium 46 (⁴⁶Ca), le calcium 48 (⁴⁸Ca), le fer 56 (⁵⁶Fe), le fer 57 (⁵⁷Fe), le fer 58 (⁵⁸Fe), le cuivre 65 (⁶⁵Cu), le zinc 66 (⁶⁶Zn), le zinc 67 (⁶⁷Zn), le zinc 68 (⁸⁶Zn), le strontium 84 (⁸⁴Sr), le strontium 87 (⁸⁷Sr), le strontium 84 (⁸⁴Sr).

4. Procédé suivant l'une quelconque des revendications précédentes, **caractérisé en ce que** l'isotope stable non radioactif est choisi parmi le deutérium, le carbone 13, l'azote 15, l'oxygène 17, l'oxygène 18, le soufre 34.

5. Procédé suivant l'une quelconque des revendications précédentes, **caractérisé en ce que** le pourcentage dudit un isotope stable non radioactif enrichi total d'au moins un élément atomique présent dans ledit composé est supérieur au pourcentage isotopique naturel.

6. Procédé suivant la revendication précédente, **caractérisé en ce que** le pourcentage isotopique de l'isotope enrichi dans ledit composé varie de 0,00014 à 100%.

7. Procédé suivant l'une quelconque des revendications précédentes, **caractérisé en ce que** l'enrichissement dudit isotope stable non radioactif se traduit dans ledit composé par un rapport teneur finale de l'isotope / teneur naturelle qui varie de 1,01 à 10⁶.

8. Procédé suivant l'une quelconque des revendications précédentes, **caractérisé en ce que** la composition contient en outre au moins un additif et/ou un adjuvant hydrophile ou lipophile choisi parmi les gélifiants; les actifs cosmétiques ou dermatologiques; les conservateurs; les antioxydants; les solvants; les parfums; les charges; les nacres; les filtres UV; les absorbeurs d'odeur; les eaux parfumées les colorants directs, les pigments minéraux, organiques ou nacrés, les précurseurs de colorant; les oxydants; les réducteurs; des agents conditionneurs pour les cheveux; des agents tensioactifs moussants anioniques, non ioniques ou amphotères; des agents de mise en forme temporaire des cheveux, les agents épaississants, les polymères filmogènes.

9. Procédé suivant l'une quelconque des revendications précédentes, **caractérisé en ce que** le dit composé est choisi parmi un solvant hydrosoluble; une silicone; un colorant capillaire; un conservateur; un polymère; un tensioactif; un agent défrisant ou de permanente; un agent oxydant; un agent réducteur; un actif cosmétique.

10. Procédé suivant la revendication précédente, **caractérisé en ce qu'**il consiste:
- à suivre le devenir et/ou les modifications dudit composé suite à son application topique sur les matières kératiniques ou les muqueuses ou,
- à mesurer l'absence de pénétration trans-cutanée ou trans-cuticulaire dudit composé, suite à son application topique sur les matières kératiniques ou les muqueuses ou,
- à suivre dans l'environnement le devenir dudit composé suite à son élimination par lavage des matières kératiniques et/ou des muqueuses.
